# DEMANDE DE BREVET EUROPEEN

(11) **EP 1 475 438 A1**
(43) Date de publication de la demande: **10.11.2004**
(21) Numéro de dépôt: 04014969.2
(22) Date de dépôt: 01.09.1997
(51) Int. Cl.: C12N 15/12, C12N 1/21, C07K 14/47, C12N 9/78, G01N 33/53

(54) **Procédé de préparation d'antigènes dérivés des filaggrines, et leur utilisation pour le diagnostic de la polyarthrite rhumatoide**

(30) Priorité: 30.08.1996 FR 9610651
(62) Demande divisionnaire de: 97938965.7
(71) Demandeur: Biomerieux, 69282 Marcy-L'Etoile (FR)
(72) Inventeur: La désignation de l'inventeur n'a pas encore été déposée
(74) Mandataire: Vialle-Presles, Marie José

(57) **Abrégé**

L'Invention est relative à un procédé de préparation d'antigène artificiel reconnu spécifiquement par les auto-anticorps anti-filaggrine présents dans le sérum de patients atteints de polyarthrite rhumatoïde, comprenant la préparation d'un polypeptide dont la séquence dérive de tout ou partie de celle d'une unité filaggrine par substitution d'au moins un résidu arginine par un résidu citrulline.

SEQUENCE LISTING

<110> BIOMERIEUX
SERRE, Guy
GIRBAL-NEUHAUSER, Elisabeth
VINCENT, Christian
SIMON, Michel
SEBBAG, Mireille
DALBON, Pascal
JOLIVET-REYNAUD, Colette
ARNAUD, Michel
JOLIVET, Michel

<120> PROCEDE DE PREPARATION D'ANTIGENES DERIVES DES FILAGGRINES, ET LEUR
UTILISATION POUR LE DIAGNOSTIC DE LA POLYARTHRITE RHUMATOIDE

<130> MJPbv1067-1

<150> 96 10651
<151> 1996-09-01

<160> 6

<170> PatentIn version 3.1

<210> 1
<211> 24
<212> DNA
<213> Artificial sequence

<220>
<223> Amorce PCR

<400> 1
ttcctatacc aggtgagcac tcat 24

<210> 2
<211> 25
<212> DNA

<213> Artificial sequence

<220>
<223> Amorce PCR

<400> 2
agaccctgaa cgtccagacc gtccc 25

<210> 3
<211> 49
<212> PRT
<213> Artificial sequence

<220>
<223> peptide S-47-S

<400> 3
Ser Thr Gly His Ser Gly Ser Gln His Ser His Thr Thr Thr Gln Gly
1               5                   10                  15
Arg Ser Asp Ala Ser Arg Gly Ser Ser Gly Ser Arg Ser Thr Ser Arg
            20                  25                  30
Glu Thr Arg Asp Gln Glu Gln Ser Gly Asp Gly Ser Arg His Ser Gly
        35                  40                  45
Ser

<210> 4
<211> 37
<212> PRT
<213> Artificial sequence

<220>
<223> Peptide S-35-R

<400> 4
Ser Gln Asp Arg Asp Ser Gln Ala Gln Ser Glu Asp Ser Glu Arg Arg
1               5                   10                  15
Ser Ala Ser Ala Ser Arg Asn His Arg Gly Ser Ala Gln Glu Gln Ser
            20                  25                  30

Arg Asp Gly Ser Arg
        35
<210> 5
<211> 14
<212> PRT
<213> Artificial sequence

<220>
<223> peptide E-12-H

<400> 5
Glu Gln Ser Ala Asp Ser Ser Arg His Ser Gly Ser Gly His
1               5                   10

<210> 6
<211> 14
<212> PRT
<213> Artificial sequence

<220>
<223> peptide E-12-D

<400> 6
Glu Ser Ser Arg Asp Gly Ser Arg His Pro Arg Ser His Asp
1               5                   10

## Description

La présente Invention est relative à de nouvelles préparations d'antigènes spécifiquement reconnus par des auto-anticorps spécifiques de la polyarthrite rhumatoïde.

La polyarthrite rhumatoïde (ci après abrégée en " PR ") est le plus fréquent des rhumatismes inflammatoires chroniques. Il s'agit d'une maladie auto-immune, et le sérum des patients atteints contient des auto-anticorps dont certains sont spécifiques, et peuvent constituer un marqueur de cette maladie, permettant son diagnostic même à des stades précoces. Des recherches ont donc été effectuées en vue d'identifier des antigènes reconnus par ces anticorps, afin d'en obtenir des préparations purifiées utilisables dans des techniques classiques de diagnostic immunologique.

Des auto-anticorps spécifiquement présents chez les malades atteints de PR et réagissant avec un antigène épithélial oesophagien de rat ont été décrits pour la première fois par B. J. J. YOUNG et al. dans Br. Med. J. 2:97-99, (1979). Ces auto-anticorps ont été à l'époque dénommés "anticorps antikératines".

Lors de précédents travaux, l'équipe des Inventeurs a obtenu, à partir d'épithéliums malpighiens humain et murin, des préparations d'antigènes apparentés à la filaggrine et à la profilaggrine, reconnus spécifiquement par les anticorps présents dans le sérum de patients atteints de polyarthrite rhumatoïde, et montré que les " anticorps antikératines " étaient en fait des auto-anticorps anti-filaggrine (ci-après dénommés " AAF "). La Demande EP 0 511 116 décrit ces préparations antigéniques, et leur utilisation pour le diagnostic de la polyarthrite rhumatoïde.

Les filaggrines sont une famille de protéines qui a été identifiée chez diverses espèces, entre autres chez l'homme, le rat, la souris, le cobaye, au niveau des épithéliums malpighiens kératinisants [pour revue sur les filaggrines, cf. DALE et al. [The Keratinocyte Handbook, Cambridge University Press, pp 323-350, (1994)]. Elles dérivent de la déphosphorylation et de la protéolyse d'un précurseur, la profilaggrine, qui est constituée essentiellement de domaines répétés de filaggrine séparés par des segments peptidiques interdomaines.

Le gène codant pour la profilaggrine se compose de sous-unités répétées dont chacune code pour une molécule de filaggrine, séparées par des portions codant pour les segments peptidiques interdomaines. Toutes les unités de répétition codant pour chacune des filaggrines humaines ont la même longueur (972 paires de base chez l'homme) ; cependant, chez l'homme, on observe des variations importantes (10-15%) de séquence d'une sous-unité à l'autre. Si la plupart sont conservatives, certaines de ces variations induisent des changements d'acides aminés et dans certains cas des changements de la charge électrique de la protéine. Ainsi les filaggrines humaines forment, indépendamment des modifications post-transcriptionnelles, une population hétérogène de molécules de taille similaire mais de séquences et de charges (pHi égal à 8,3 ± 1,1) différentes [GAN et al., Biochem. 29, p. 9432-9440 (1990)].

La profilaggrine est une protéine de poids moléculaire élevé (environ 400 000 chez l'homme) soluble en présence de fortes concentrations de sels ou d'urée. Elle possède une forte teneur en acides aminés basiques (arginine et histidine), ainsi qu'en glycine, sérine et acide glutamique. Elle est pauvre en acides aminés non polaires et ne contient ni méthionine, ni cystéine, ni tryptophane. Elle est fortement phosphorylée sur des résidus sérine, ce qui lui confère un point isoélectrique proche de la neutralité.

La profilaggrine est clivée en unités filaggrine au cours d'un processus complexe de maturation, impliquant une déphosphorylation, suivie d'un clivage par des protéases au niveau des segments interdomaines. Ce clivage génère d'abord des fragments de taille intermédiaire, puis les molécules fonctionnelles de filaggrine.

Les filaggrines issues de la déphosphorylation et du clivage de la profilaggrine sont des protéines basiques dont le contenu en acides aminés est similaire à celui des profilaggrines. Elles participent à l'organisation des filaments de kératine, et subissent une maturation progressive au cours de laquelle les résidus arginine, basiques, sont convertis en résidus citrulline, neutres, sous l'action de la peptidylarginine déiminase [HARDING C.R. et SCOTT I.R., J. Mol. Biol. 170, p. 651-673 (1983)]. Ceci entraîne une réduction de leur affinité pour les kératines dont elles se détachent ; elles sont alors totalement dégradées sous l'action de diverses protéases.

Les propriétés des filaggrines et des profilaggrines ont été particulièrement bien étudiées chez le rat, chez la souris et chez l'homme. La taille de la profilaggrine varie, selon les espèces, de 300 à 400 kD et celle des filaggrines de 27 à 64 kD.

Le polymorphisme observé chez l'homme entre les séquences des unités filaggrine à l'intérieur d'un même gène de profilaggrine n'apparaît pas chez le rat et la souris. Les filaggrines présentent en outre une grande variabilité inter et intra-spécifique au niveau de leur séquence. Cette variabilité n'affecte toutefois pas leurs propriétés fonctionnelles, ni leur composition globale en acides aminés, et leurs propriétés biochimiques. De même, les localisations tissulaires de la profilaggrine et des filaggrines sont identiques chez les différents mammifères étudiés.

En poursuivant leurs travaux, les Inventeurs ont constaté que la profilaggrine présente dans les granules de kératohyaline de l'épiderme humain n'était contrairement aux filaggrines, pas reconnue par les AAF[SIMON et al. Clin. Exp. Immunol. 100, 90-98 (1995)]. Ils ont alors testé la réactivité des AAF avec de la filaggrine recombinante, et ont constaté que celle-ci non plus n'était pas reconnue. D'autre part, il avait été précédemment observé que les formes des filaggrines épidermiques humaines principalement reconnues par les AAF étaient les formes acido-neutres décrites par SIMON et al. [J. Clin. Invest., 92, 1387, (1993)] et dans la demande EP 0 511 116. Le fait que ces formes acido-neutres correspondent à un stade tardif de maturation de la filaggrine, permettait de supposer que tout ou partie des modifications post-traductionnelles intervenant jusqu'à ce stade étaient impliquées dans la formation des épitopes reconnus par les AAF.

Pour vérifier cette hypothèse, les Inventeurs ont cherché à reproduire *in vitro,* à partir de filaggrine recombinante, ces modifications post-traductionnelles, afin de déterminer lesquelles étaient susceptibles d'influer sur l'antigénicité de la filaggrine.

Ils ont ainsi constaté qu'en fait, la citrullination de la filaggrine suffisait à générer des épitopes reconnus par les AAF. En effet, ils ont observé, en procédant à la déimination *in vitro* de filaggrine recombinante que le remplacement d'au moins une partie des arginines par des citrullines permet l'obtention d'un antigène reconnu spécifiquement par les AAF présents dans le sérum des patients atteints de PR.

La présente Invention a pour objet un antigène artificiel reconnu spécifiquement par les AAF présents dans le sérum des patients atteints de PR, caractérisé en ce qu'il est constitué par un polypeptide recombinant ou de synthèse, comprenant tout ou partie d'une séquence dérivée de celle d'une unité filaggrine ou d'une molécule apparentée, par substitution d'au moins un résidu arginine par un résidu citrulline. De préférence, un antigène conforme à l'invention comprend au moins 5 acides aminés consécutifs, et avantageusement au moins 10 acides aminés consécutifs, dont au moins une citrulline, de ladite séquence.

Au sens de la présente Invention, on entend par : unité filaggrine ", un polypeptide dont la séquence est celle du produit de traduction de l'une quelconque des sous-unités codant pour un domaine filaggrine du gène de la profilaggrine humaine ou d'une autre espèce, ou bien est une séquence consensus, séquence théorique obtenue à partir des séquences des domaines filaggrine.

Au sens de la présente Invention, on entend par : " molécule apparentée " toute molécule ayant au moins un résidu arginine susceptible d'être transformé en résidu citrulline sous l'action d'une PAD (peptidyl arginine déiminase) ; à titre d'exemple, cette PAD peut être une PAD de muscle de lapin, comme le montrent les exemples ci-après. Il est cependant à la portée de l'homme de l'art de sélectionner toute autre PAD appropriée par de simples essais de routine, en la faisant réagir avec la filaggrine humaine non-citrullinée.

Le terme "peptide" tel qu'utilisé dans la présente Demande signifie notamment protéine ou fragment de protéine, oligopeptide, extrait, séparé ou substantiellement isolé ou synthétisé, notamment ceux obtenus par synthèse chimique ou par expression dans un organisme recombinant ; tout peptide dans la séquence duquel un ou plusieurs acides aminés de la série L sont remplacés par un acide aminé de la série D, et vice-versa ; tout peptide dont l'une au moins des liaisons CO-NH, et avantageusement toutes les liaisons CO-NH de la chaîne peptidique est (sont) remplacée(s) par une (des) liaisons NH-CO ; tout peptide dont l'une au moins des liaisons CO-NH et avantageusement toutes les liaisons CO-NH est ou sont remplacée(s) par une ou des liaison(s) NH-CO, la chiralité de chaque résidu aminoacyle, qu'il soit impliqué ou non dans une ou plusieurs liaisons CO-NH susmentionnées, étant soit conservée, soit inversée par rapport aux résidus aminoacyles constituant un peptide de référence, ces composés étant encore désignés immunorétroïdes, un mimotope, etc.

Des antigènes conformes à l'invention peuvent par exemple être obtenus par action de la PAD sur des protéines ou des peptides naturels, recombinants, ou de synthèse, comportant des résidus arginine ; ils peuvent également être obtenus par synthèse peptidique, en incorporant directement un ou plusieurs résidus citrulline dans le peptide synthétisé.

Selon un mode de réalisation préféré d'un antigène conforme à la présente Invention, il est constitué par un polypeptide comprenant tout ou partie de la séquence correspondant aux acides aminés 144 à 314 d'une unité filaggrine humaine, dans laquelle au moins un résidu arginine a été substitué par un résidu citrulline, ou bien tout ou partie de la séquence correspondant aux acides aminés 76 à 144 d'une unité filaggrine humaine, dans laquelle au moins un résidu arginine a été substitué par un résidu citrulline.

Un antigène conforme à l'invention peut par exemple être constitué par un peptide comprenant tout ou partie de la séquence correspondant aux acides aminés 71 à 119 d'une unité de filaggrine humaine, dans laquelle au moins un résidu arginine a été substitué par un résidu citrulline.

Avantageusement, un antigène conforme à l'invention est constitué par un peptide comprenant tout ou partie d'au moins une séquence dérivée de l'une des séquences identifiées dans la liste de séquences en annexe sous les numéros SEQ ID NO: 3, SEQ ID NO: 5, SEQ ID NO: 6, par substitution d'au moins un résidu arginine par un résidu citrulline.

La présente Invention a également pour objet l'utilisation des antigènes conformes à l'invention, tels que définis ci-dessus, pour le diagnostic *in vitro* de la PR.

La présente invention englobe en particulier des compositions antigéniques pour le diagnostic de la présence d'auto-anticorps spécifiques de la PR dans un échantillon biologique, lesquelles compositions sont caractérisées en ce qu'elles contiennent au moins un antigène conforme à l'invention, éventuellement marqué et/ou conjugué avec une molécule porteuse.

La présente Invention a également pour objet un procédé de détection des auto-anticorps de classe G spécifiques de la PR dans un échantillon biologique, lequel procédé est caractérisé en ce qu'il comprend :
- la mise en contact dudit échantillon biologique avec au moins un antigène conforme à l'Invention, tel que défini ci-dessus, dans des conditions permettant la formation d'un complexe antigène/anticorps avec les auto-anticorps spécifiques de la PR éventuellement présents ;
- la détection, par tous moyens appropriés, du complexe antigène/anticorps éventuellement formé.

Ce procédé de détection peut être mis en oeuvre grâce à un nécessaire comprenant au moins un antigène selon l'Invention, ainsi que des tampons et réactifs appropriés pour la constitution d'un milieu réactionnel permettant la formation d'un complexe antigène/anticorps, et/ou des moyens de détection dudit complexe antigène/anticorps

Ledit nécessaire peut également comprendre, le cas échéant, des échantillons de référence, tels qu'un ou plusieurs sérum(s) négatif(s) et un ou plusieurs sérum(s) positif(s).

La présente invention sera mieux comprise à l'aide du complément de description qui va suivre, qui se réfère à des exemples de préparation et de mise en oeuvre d'antigènes conformes à l'invention.

### EXEMPLE 1 : REACTIVITE DES SERUMS PROVENANT DE PATIENTS ATTEINTS DE POLYARTHRITE RHUMATOIDE SUR LES FILAGGRINES EPIDERMIQUES

On broie à l'aide d'un broyeur de type " potter " électrique un lambeau d'épiderme humain dans un tampon à haute concentration en urée (6M), ce qui permet de solubiliser l'ensemble des filaggrines épidermiques.

Avec cet extrait épidermique, on réalise une électrophorèse bidimensionnelle (gel 8-25% d'acrylamide, en présence d'urée 6M); la 1ère dimension correspond à une isoélectrofocalisation sur gel dans un gradient de pH allant de 5 à 8 et la seconde dimension correspond à une électrophorèse en conditions dénaturantes, en présence de SDS. Après électrophorèse, les protéines du gel sont transférées sur nitrocellulose.

Les réactions immunologiques sont effectuées selon un protocole classique.

La membrane de nitrocellulose est incubée pendant une nuit à 4°C avec un sérum de patient atteint de PR, dilué au 1/2000, puis les immunoglobulines du sérum qui ont réagi avec les antigènes fixés sur la membrane sont détectées à l'aide d'un anticorps secondaire anti-IgG humaines marqué à la peroxydase. La révélation en présence du substrat de la peroxydase est effectuée par la méthode ECL (Enhanced Chemi-Luminescence, AMERSHAM) selon le protocole préconisé par le fabricant.

Dans un deuxième temps, la même membrane est lavée puis incubée pendant 1 heure et demie à 20°C, en présence cette fois de l'anticorps monoclonal AHF1 décrit par SIMON et al. [J. Invest. Dermatol. 105, 432, (1995)] à une concentration de 0,2 µg/ml, puis d'un anticorps secondaire anti-IgG de souris marqué à la peroxydase. La réaction est révélée par la méthode ECL, comme indiqué ci-dessus.

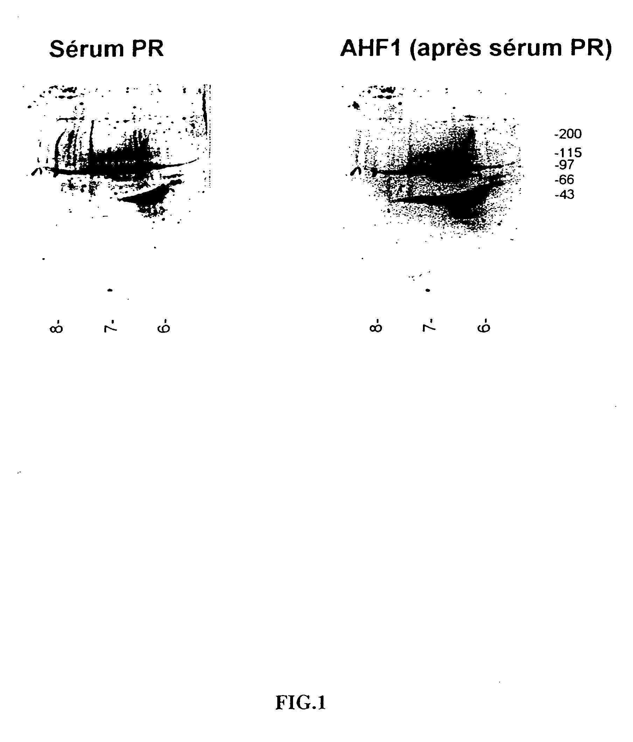

Les résultats sont illustrés par la figure 1 :

L'anticorps monoclonal AHF1 reconnaît des

isoformes de filaggrine dont le pHi s'échelonne de 5,8 à 8,5. En revanche, seules les isoformes dont le pHi s'échelonne de 5,8 à 7,4, sont détectées par le sérum du patient atteint de PR

Le fait que seules les isoformes les plus acides de filaggrines soient détectées, permet de supposer que l'acidification de ces isoformes fait partie des modifications post-traductionnelles qui seraient nécessaires à la reconnaissance de la filaggrine par les anticorps présents dans les sérums des patients atteints de PR.

### EXEMPLE 2 : DEIMINATION IN VITRO DE FILAGGRINE RECOMBINANTE PAR LA PEPTIDYL ARGININE DEIMINASE (P.A.D.).

De la filaggrine recombinante est produite selon le protocole suivant :

Un fragment d'ADN codant pour une unité filaggrine est amplifié par PCR, à partir d'ADN génomique humain (cellules RAJI : ATCC CCL86) à l'aide des 2 amorces suivantes :

Amorce 5' :
5' TTCCTATACCAGGTGAGCACTCAT 3'

Amorce 3' :
5' AGACCCTGAACGTCCAGACCGTCCC 3'

Le produit d'amplification est cloné dans le site SmaI du vecteur pUC19. On procède à la sélection des clones recombinants, en vérifiant la présence d'un insert de 972 pb obtenu après digestion avec SacI et XbaI. Cet insert est ensuite sous-cloné dans pUC19. L'insert résultant de ce sous-clonage est ensuite transféré dans le vecteur pGEX (commercialisé par la société PHARMACIA), entre les sites EcoRI et HindIII. Le vecteur d'expression ainsi obtenu exprime, dans *E. coli,* la filaggrine en fusion avec la glutathion-S-transférase (GST), sous contrôle du promoteur procaryote Tac. La synthèse de la protéine recombinante est induite par addition d'isopropyl-β-D-galactoside (IPTG) à la culture.

La filaggrine recombinante ainsi obtenue sera dénommée ci-après : " fil-gst ".

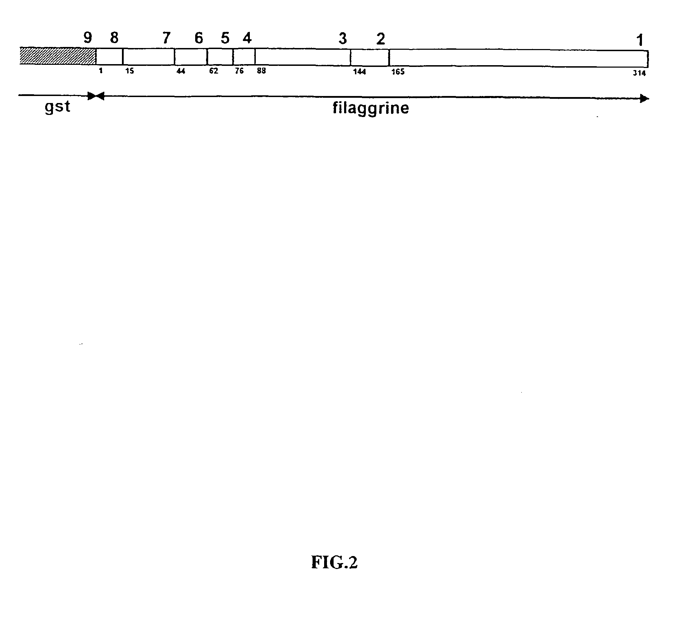

On constate après électrophorèse, l'existence de 9 fragments qui résultent d'une protéolyse post-traductionnelle de la filaggrine entière. Les emplacements des différentes coupures générant ces fragments sont indiqués sur la figure 2.

Le mélange des 9 fragments est soumis à une déimination *in vitro* par la peptidyl arginine déiminase.

On utilise une préparation de peptidyl arginine déiminase de muscle de lapin (681 U/ml) commercialisée par TAKARA BIOMED EUROPE, selon le protocole préconisé par le fabricant.

Les conditions opératoires sont les suivantes :
- Milieu réactionnel : Tris-HCl 0,1 M, CaCl₂ 10 mM, DTT 5 mM, pH 7,4 ;
- Rapport enzyme/substrat : 140 mU/µmole de filaggrine contenant 10% d'arginine soit 4 mU/µmole d'arginine ;
- Incubation : entre 0 et 60 mn à 50°C ;
- Arrêt de la réaction : chauffage 3 mn en tampon de LAEMMLI

On effectue en parallèle les 8 réactions suivantes.
(1) BSA (sérum albumine bovine) incubée dans milieu réactionnel (1h, 50°C) sans P.A.D.
(2) BSA incubée dans milieu réactionnel (1h, 50°C) avec 60 mU de P.A.D.
(3) fil-gst incubée dans milieu réactionnel (1h, 50°C) sans P.A.D.
(4) fil-gst incubée dans milieu réactionnel (5 minutes à 50°C) avec 60 mU de P.A.D.
(5) fil-gst incubée dans milieu réactionnel (15 minutes à 50°C) avec 60 mU de P.A.D.
(6) fil-gst incubée dans milieu réactionnel (30 minutes à 50°C) avec 60 mU de P.A.D.
(7) fil-gst incubée dans milieu réactionnel (1h à 50°C) avec 60 mU de P.A.D.
(8) fil-gst incubée dans milieu réactionnel (1h à 50°C) avec 60 mU de P.A.D. et en présence de 10 mM de N-éthylmaléimide (inhibiteur de la P.A.D.).

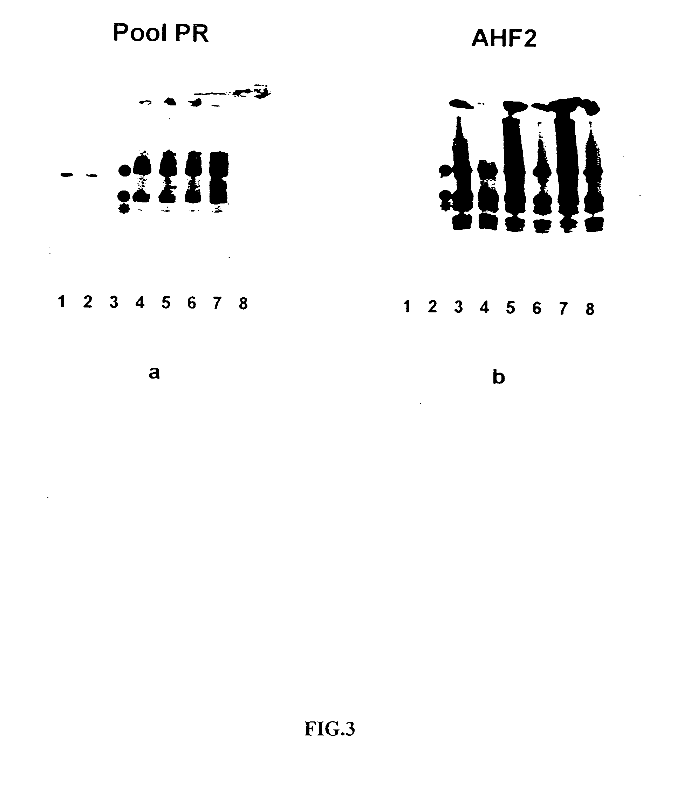

On dépose 1 µl de chaque échantillon sur un gel d'électrophorèse (gel PHAST®-SDS 12,5%, PHARMACIA), et on réalise l'électrophorèse avec l'appareil PHAST-SYSTEM® (PHARMACIA), dans les conditions préconisées par le fabricant. Après transfert sur nitrocellulose, on révèle soit avec un pool de 5 sérums de patients atteints de PR , dilué au 1/2000 (Figure 3a), soit avec l'anticorps monoclonal anti-filaggrine AHF2 [SIMON et al. J. Invest. Dermatol. 105, 432, (1995)] à la concentration de 0,2 µg/ml(Figure 3b).

Le complexe antigène/anticorps est révélé à l'aide d'un anticorps secondaire couplé à la peroxydase, par la technique ECL.

Les résultats sont illustrés par la figure 3
Piste 1 : BSA (1 heure, 50°C)
Piste 2 : BSA + PAD (1 heure, 50°C)
Piste 3 : fil-gst (1 heure, 50°C)
Piste 4 : fil-gst + PAD (5 minutes, 50°C)
Piste 5 : fil-gst + PAD(15 minutes, 50°C)
Piste 6 : fil-gst + PAD (30 minutes, 50°C)
Piste 7 : fil-gst + PAD (1 heure, 50°C)
Piste 8 : fil-gst + PAD + inhibiteur. (1 heure, 50°C)

En l'absence de réaction de citrullination, la fil-gst n'est pas reconnue par les sérums de patients atteints de PR (Figure 3a, piste 3), alors que dès 5 minutes de citrullination (Figure 3a, piste 4), elle est détectée par ces sérums. On constate une augmentation de la réactivité avec le pool de sérums quand on fait agir la P.A.D. pendant 60 minutes à 50°C (Figure 3a, piste 7).
- les fragments 1, 2, 3 (bandes repérées par des points) de la fil-gst sont fortement reconnus, après citrullination, par les sérums de patients atteints de PR. Les fragments 4 et 5 (bandes repérées par des astériques) sont également reconnus. Ces résultats permettent de supposer qu'il existe un ou plusieurs épitopes de forte affinité dans la moitié COOH-terminale de la filaggrine (144 à 314), cet épitope étant répété entre les positions 76 et 144.
- l'anticorps monoclonal AHF2 reconnaît tous les fragments de fil-gst, citrullinée ou non.

### EXEMPLE 3 : SPECIFICITE DE LA RECONNAISSANCE DE LA FIL-GST CITRULLINEE PAR LES SERUMS

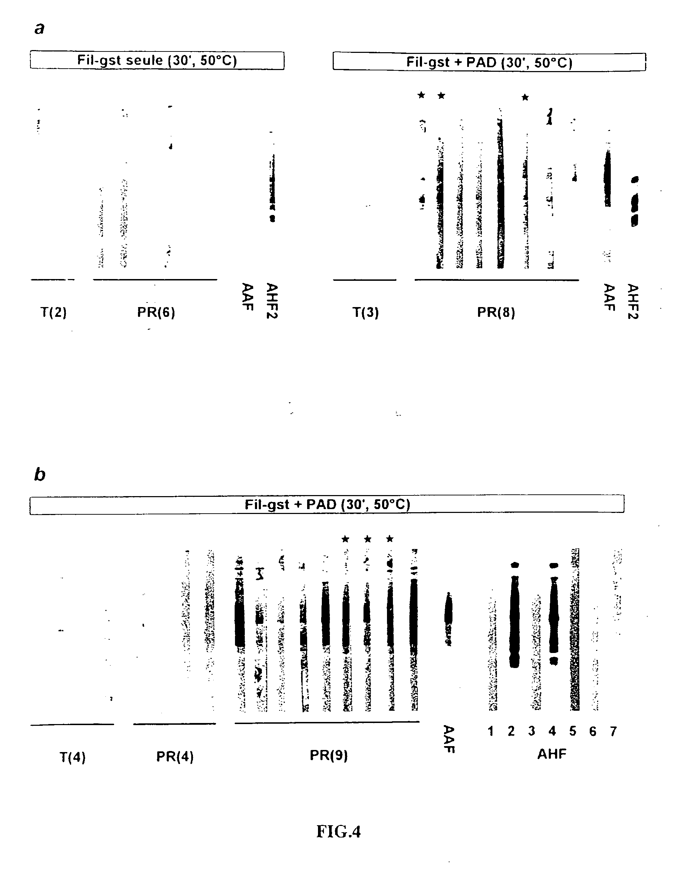

Dans une première série d'expériences, (Figure 4a) on compare la réactivité de la fil-gst non citrullinée (fil-gst seule, 30 minutes à 50°C) et de la fil-gst citrullinée par la P.A.D. 30 minutes à 50°C avec des sérums humains composés de :
- sérums de personnes normales : T(2) et T(3)
- sérums de patients atteints de PR présentant des titres élevés en AAF détectés par immunotransfert sur les variants acido-neutres de la filaggrine humaine, et par immunofluorescence indirecte sur cryocoupes d'oesophage de rat : PR(6)et PR(8).
- anticorps anti-filaggrine purifiés à partir du sérum d'un patient atteint de PR par chromatographie d'affinité, sur une colonne greffée avec les isoformes acido-neutres de la filaggrine humaine : AAF.

Un contrôle positif est aussi réalisé avec l'anticorps monoclonal AHF2.

Dans une seconde série d'expériences (Figure 4b), on confirme la réactivité de la fil-gst citrullinée avec une plus large série de sérums :
- 4 sérums témoins : T(4)
- 4 sérums de patients atteints de PR ne présentant pas d'AAFs détectables en immuno-transfert ou en immunofluorescence indirecte : PR(4)
- 9 sérums de patients atteints de PR avec des titres élevés en AAF (trois d'entre eux (*) ont été aussi testés dans la première série d'expérimentations) :PR(9)
- des anticorps anti-filaggrine purifiés par chromatographie d'affinité, sur une colonne greffée avec les isoformes acido-neutres de la filaggrine, à partir d'un pool de sérums de 40 patients atteints de PR : AAF
- les anticorps monoclonaux AHF (1-7).

Les sérums sont utilisés à la dilution de 1/2000 ; les anticorps anti-filaggrine purifiés par chromatographie d'affinité sont utilisés à la concentration de 4 µg/ml ; les anticorps monoclonaux sont utilisés à la concentration de 0,2 µg/ml

Les résultats sont les suivants :
- La citrullination de la filaggrine recombinante est nécessaire pour la reconnaissance par les AAFs des sérums de patients atteints de PR (14 sérums positifs sur 14 la reconnaissent) ;
- les auto-anticorps anti-filaggrine, purifiés par chromatographie d'affinité à partir des sérums de patients atteints de PR, montrent la même réactivité sur fil-gst citrullinée que les sérums de patients atteints de PR (reconnaissance des fragments correspondant aux bandes 1 à 5). Ceci montre que ce sont bien les AAF présents dans ces sérums qui reconnaissent la fil-gst citrullinée.

### EXEMPLE 4 : CITRULLINATION DES PEPTIDES S-47-S ET S-35-R PAR LA P.A.D, ET ESSAI DE LA REACTIVITE DES PEPTIDES CITRULLINES.

Le peptide de 49 acides aminés S-47-S de séquence (code 1 lettre) :
NH₂-STGHSGSQHSHTTTQGRSDASRGSSGSRSTSRETRDQEQSGDGSRHSGS-COOH
correspondant aux acides aminés 71 à 119 de la séquence d'une unité de filaggrine humaine, et comportant 6 résidus arginine, et
le peptide de 37 acides aminés S-35-R de séquence (code 1 lettre) :
NH₂-SQDRDSQAQSEDSERRSASASRNHRGSAQEQSRDGSR-COOH
correspondant aux acides aminés 155 à 191 de la séquence d'une unité de filaggrine humaine, et comportant 7 résidus arginine, ont été préparés par synthèse peptidique. Les peptides S-47-S et S-35-R sont représentés dans la liste de séquences en annexe sous les numéros respectifs SEQ ID NO: 3 et SEQ ID NO: 4.

Ces 2 peptides, ainsi que la fil-gst, ont été citrullinés par action de la P.A.D., pendant 30 minutes à 50°C, dans le même milieu réactionnel que celui indiqué à l'exemple 2. Les conditions spécifiques pour chaque peptide, et pour la fil-gst sont les suivantes :
- peptide S-47-S : 4 mU/µmole arginine
- peptide S-35-R : 2,7 mU/µmole arginine
- fil-gst : comme indiqué à l'exemple 2.

On compare, par " dot-blot ", la réactivité de chaque peptide et celle de la fil-gst, avant et après action de l'enzyme, vis-à-vis de l'anticorps monoclonal AHF4, et du sérum d'un patient atteint de PR.

Les conditions opératoires sont les suivantes :
- 0,5 µg par dépôt de chaque antigène (peptides, fil-gst, variants acido-neutres de la filaggrine (VAF))
- Traitement de la nitrocellulose 45 minutes à 80°C, avant immunodétection.
- sérum PR utilisé à la dilution de 1/2000 ; anticorps monoclonal AHF4 utilisé à une concentration de 0,2 µg/ml

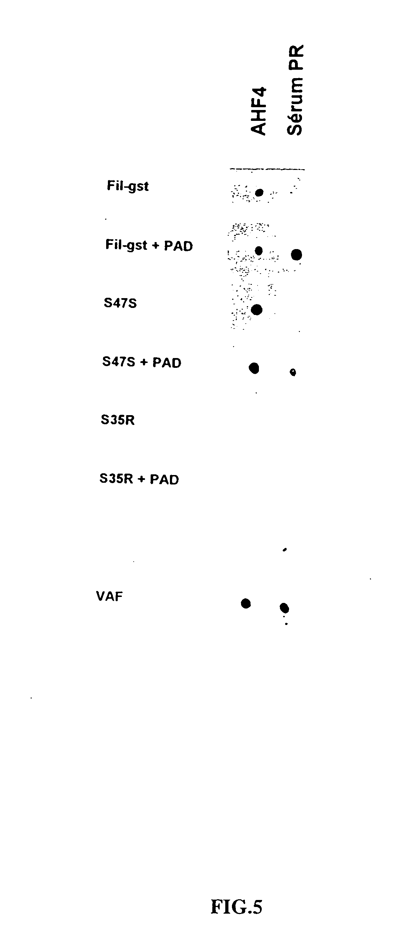

Les résultats sont illustrés par la Figure 5, qui montre que :
- AHF4 reconnaît le peptide S-47-S et la fil-gst citrullinés ou non mais ne reconnaît pas S-35-R, citrulliné ou non.
- S-47-S est reconnu, après citrullination, par le sérum du patient atteint de PR, alors que S-35-R, citrulliné ou non, n'est pas reconnu. Le même sérum reconnaît par ailleurs les VAF et la fil-gst citrullinée, mais ne reconnaît pas la fil-gst non-citrullinée.

### EXEMPLE 5 : SYNTHESE DES PEPTIDES E-12-H ET E-12-D CITRULLINES ET NON CITRULLINES ET ESSAI DE LA REACTIVITE DES PEPTIDES.

Les peptides E-12-H et E-12-D ont été déterminés par référence aux séquences nucléotidiques du gène de la profilaggrine humaine décrites par GAN S.Q et al. [Biochemistry, 29 : 9432-9440, (1990)].

Le peptide de 14 acides aminés E-12-H de séquence (code 1 lettre) :
NH₂-EQSADSSRHSGSGH-COOH
comprend 1 résidu arginine, et
le peptide de 14 acides aminés E-12-D de séquence (code 1 lettre) :
NH₂-ESSRDGSRHPRSHD-COOH
comprend 3 résidus arginine.

Les peptides E-12-H et E-12-D sont représentés dans la liste de séquences en annexe sous les numéros respectifs SEQ ID NO: 5 et SEQ ID NO: 6.

Ces peptides ont été préparés par synthèse peptidique en phase solide.

Les peptides E-12-H et E-12-D citrullinés ont été synthétisés directement par incorporation d'une citrulline en remplacement d'une arginine.

Pour le peptide E-12-D, seul le résidu arginine correspondant au 8^{ème} acide aminé de la séquence a été remplacé par une citrulline lors de la synthèse peptidique.

La réactivité de chaque peptide citrulliné et non citrulliné a été testée respectivement vis-à-vis d'un sérum normal, de deux sérums de patients PR, d'anticorps anti-filaggrine (AFAs) purifiés à partir d'un pool de 45 sérums de patients PR et d'anticorps anti-filaggrine purifiés à partir de 12 sérums de patients PR.

### PROTOCOLE EXPERIMENTAL :

Les puits de plaques de microtitration NUNC MAXISORP ont été revêtus respectivement à l'aide des peptides E-12-D et E-12-H non-citrullinés et citrullinés, dilués à une concentration de 5 µg/ml dans un tampon PBS (pH: 7,4) et incubés pendant une nuit à 4°C (volume final : 100 µg/puits). Les puits ont été saturés pendant 30 minutes à 37°C en PBS-Tween 20, 0,05% gélatine 2,5%, 200 µl/puits. Le sérum de contrôle négatif (sérum normal) a été dilué au 1/120. Les anticorps anti-filaggrine ont été dilués en PBS-Tween 20, 0,05%-gélatine 0,5% (PBS TG) de sorte que les concentrations finales en auto-anticorps anti-filaggrine soient celles indiquées dans le tableau I annexé. Le sérum de contrôle négatif, les sérums PR et les anticorps anti-filaggrine ont été ajoutés (volume final : 100 µl/puits) et soumis à incubation pendant 1 heure à 37°C et une nuit à 4°C. Des anticorps de chèvre anti-chaînes lourdes gamma des immunoglobulines humaines, marqués à la peroxydase (commercialisés par la société SOUTHERN BIOTECHNOLOGIES) ont été ajoutés dans chaque puits (dilution en PBSTG : 1/2000, volume final : 100 µl/puits) et soumis à incubation pendant 1 heure à 37°C. La révélation a été effectuée par addition d'orthophénylènediamine (2mg/ml, pendant 10 minutes).

Les résultats présentés dans le tableau I annexé sont donnés en rapport de DO à 492 nm : signal peptide citrulliné/signal peptide non-citrulliné.

Ces résultats montent que, dans la majorité des cas, le rapport en DO peptide citrulliné/peptide non-citrulliné est supérieur à 1, et illustrent donc la bonne sensibilité des peptides citrullinés par rapport aux peptides non-citrullinés pour leur réactivité vis-à-vis des autoanticorps anti-filaggrine.

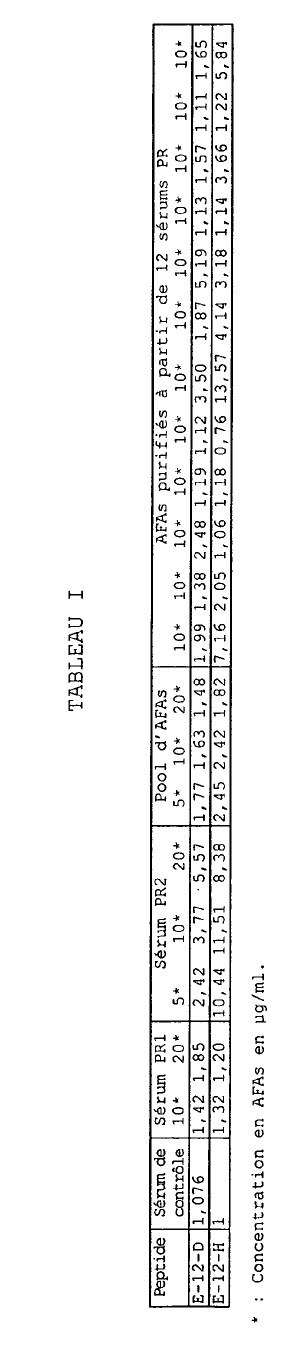

## Revendications

1. Procédé d'obtention d'un antigène artificiel utilisable pour le diagnostic *in vitro* de la polyarthrite rhumatoïde, **caractérisé en ce qu'**il comprend la préparation d'un polypeptide recombinant ou de synthèse, dont la séquence est dérivée de celle de tout ou partie d'une unité filaggrine par substitution d'au moins un résidu arginine par un résidu citrulline, et qui est reconnu spécifiquement par les auto-anticorps anti-filaggrine présents dans le sérum de patients atteints de polyarthrite rhumatoïde.

2. Procédé selon la revendication 1, **caractérisé en ce que** la séquence dudit polypeptide est dérivée de tout ou partie d'une séquence choisie parmi :
- la séquence correspondant aux acides aminés 144 à 314 d'une unité filaggrine humaine, ou bien
- la séquence correspondant aux acides aminés 76 à 144 d'une unité filaggrine humaine,
par substitution d'au moins un résidu arginine par un résidu citrulline.

3. Procédé selon la revendication 2, **caractérisé en ce que** la séquence dudit polypeptide est dérivée de tout ou partie d'une séquence correspondant aux acides aminés 71 à 119 d'une unité de filaggrine humaine, par substitution d'au moins un résidu arginine par un résidu citrulline.

4. Procédé selon la revendication 1, **caractérisé en ce que** la séquence dudit polypeptide est dérivée de tout ou partie d'une séquence choisie parmi les séquences SEQ ID NO: 3, SEQ ID NO: 5, SEQ ID NO: 6, par substitution d'au moins un résidu arginine par un résidu citrulline.

5. Procédé selon une quelconque des revendications 1 à 4, **caractérisé en ce que** la substitution d'au moins un résidu arginine par un résidu citrulline est effectuée par l'action d'une peptidyl arginine déiminase.

6. Procédé selon une quelconque des revendications 1 à 4, **caractérisé en ce que** la substitution d'au moins un résidu arginine par un résidu citrulline est effectuée par incorporation dudit ou desdits résidu(s) citrulline dans ledit peptide lors de sa synthèse.

7. Procédé de préparation d'une composition antigénique pour le diagnostic de la présence d'auto-anticorps spécifiques de la polyarthrite rhumatoïde dans un échantillon biologique, **caractérisée en ce qu'**il comprend :
- la préparation d'un antigène artificiel par un procédé selon une quelconque des revendications 1 à 6 ;
- l'incorporation dudit antigène dans ladite composition.

8. Procédé selon la revendication 7, **caractérisé en ce qu'**il comprend en outre le marquage dudit antigène, et/ou sa conjugaison avec une molécule porteuse.

9. Procédé de préparation d'un nécessaire pour la détection des auto-anticorps spécifiques de la polyarthrite rhumatoïde dans un échantillon biologique, **caractérisé en ce qu'**il comprend :
- la préparation d'un antigène artificiel par un procédé selon une quelconque des revendications 1 à 6 ;
- l'association dudit antigène avec des tampons et réactifs appropriés pour la constitution d'un milieu réactionnel permettant la formation d'un complexe antigène/anticorps, et/ou avec des moyens de détection dudit complexe antigène/anticorps.
